# EUROPEAN PATENT APPLICATION

(11) **EP 1 825 877 A1**
(43) Date of publication of application: **29.08.2007**
(21) Application number: 05806974.1
(22) Date of filing: 17.11.2005
(51) Int. Cl.: A61M 5/145

(54) **CHEMICAL LIQUID INFUSION SYSTEM**

(30) Priority: 18.11.2004 JP 2004334942
(71) Applicant: Nemoto Kyorindo Co., Ltd., Tokyo 113-0033 (JP)
(72) Inventor: NEMOTO, Shigeru, c/o Nemoto Kyorindo Co., Ltd., Tokyo 1130033 (JP); ONO, Seiichi, c/o Nemoto Kyorindo Co., Ltd., Tokyo 1130033 (JP); SAKAKIBARA, Masahiro, Nemoto Kyorindo Co., Ltd., Tokyo 1130033 (JP)
(74) Representative: Burbaud, Eric
(86) International application number: PCT/JP2005/021132
(87) International publication number: WO 2006/054651

(57) **Abstract**

A chemical solution injection system, wherein a RFID chip (230) is mounted on a chemical liquid syringe (200) in the state of being wrapped around the outer surface of the cylinder member (210) thereof. An amplification antenna (240) wrapped one turn around the outer surface of the cylinder member (210) is disposed at the front of the RFID chip (230) parallel with each other. Since the communication performance of the RFID chip is increased by the amplifying antenna, the RFID chip (230) of the chemical solution syringe (200) is allowed to satisfactorily communicate with the RFID reader (131) of injection head (110).

## Description

### Technical Field

The present invention relates to a chemical solution injection system for injecting a chemical solution of chemical solution syringe into a patient by using a chemical solution injector, and more particularly, to a chemical solution injection system for injecting a contrast medium into a patient whose diagnostic images are taken by an imaging diagnostic apparatus such as a CT (Computed Tomography) scanners.

### Background Art

Presently available imaging diagnostic apparatuses for capturing diagnostic images of patients include CT scanners, MRI (Magnetic Resonance Imaging) apparatuses, PET (Positron Emission Tomography) apparatuses, ultrasonic diagnostic apparatuses, CT angiography apparatuses, MRA (MR angiography) apparatuses and the like. When the abovementioned imaging dia gnostic apparatuses are used, a chemical solution such as a contrast medium and physiological saline may be injected into a patient. Chemical solution injectors for automatically performing the injection have been put into practical use.

Such a chemical solution injector has a piston driving mechanism formed of a driving motor, a slider mechanism and the like, for example. A chemical solution syringe is removably mounted on the injector. The chemical solution syringe typically includes a cylinder member and a piston member slidably inserted in the cylinder member.

More specifically, the cylinder member is formed in a cylindrical shape and has a front end closed and having a conduit formed at the center thereof and a rear end opened. The cylinder member has an annular cylinder flange formed on the outer circumference of the rear end, and the piston member is slidably inserted into the cylinder member through the opening at the rear end.

There are a pre-filled type and a refill type in the chemical solution syringe. The chemical solution syringe of the pre-filled type includes a cylinder member filled with a chemical solution and is wholly sealed by a packing material for shipment. The chemical solution syringe of the refill type includes a cylinder member which can be filled with a desired liquid by a user. For simplicity, the following description will be made assuming that the chemical solution syringe of the pre-filled type is used.

When the chemical solution in the chemical solution syringe of the abovementioned type is injected into a patient, an operator prepares for a chemical solution syringe containing an appropriate chemical solution and takes out the chemical solution syringe from the packing material. The operator connects the chemical solution syringe to a patient through an extension tube and mounts the chemical solution syringe on a chemical solution injector. The cylinder flange thereof is held by a cylinder holding mechanism. In this state, the chemical solution injector presses the piston member into the cylinder member with the piston driving mechanism in accordance with a predetermined operation to inject the chemical solution into the patient from the syringe.

The operator determines the speed at which the chemical solution is injected and the total quantity of the chemical solution to be injected in view of the type of the chemical solution and the like, and enters data representing the speed and total quantity into the chemical solution injector. The chemical solution injector injects the chemical solution into the patient based on the entered data. For example, if a contrast medium is injected as the chemical solution, the image contrast of the patient is changed to allow the imaging diagnostic apparatus to capture a favorable diagnostic image of the patient.

Some chemical solution injectors can inject physiological saline as well as the contrast medium into the patient. In such a chemical solution injector, the operator enters as desired an instruction to inject the physiological saline following the completion of the injection of the contrast medium, together with data representing the injection speed and total quantity of the physiological saline, into the chemical solution injector. Based on the entered data, the chemical solution injector first injects the contrast medium into the patient based on the entered data and then automatically injects the physiological saline. The subsequently injected physiological saline can push the previously injected contrast medium to reduce the consumption of the contrast medium and also can reduce artifacts in the captured image.

In the chemical solution injector as described above, it is necessary to enter various types of data about the chemical solution and the chemical solution syringe in order to appropriately perform injection operation. This entering operation is cumbersome. To address this, a chemical solution injection system has been proposed, in which an RFID chip is put on a chemical solution syringe, and an RFID reader is mounted on a chemical solution injector, to transmit various types of data automatically from the chemical solution syringe to the chemical solution injector (see, for example, Japanese Patent No. 3323204).

In the chemical solution injection system disclosed in the above patent, air coils serving as amplification antennas are individually placed on a pair of convex portions of a cylinder flange of a chemical solution syringe, and an RFID chip measuring several millimeters per side is put on one of the pair of concave portions, and the RFID chip is connected to the pair of air coils.

In many chemical solution syringes commercially available in recent years, a cylinder flange protrudes from the outer circumference surface of a cylinder member only approximately 3 to 4 mm. It is extremely difficult to place the RFID chip on such a cylinder flange, and it is more difficult to place the air coils there.

A currently used RFID chip is typically formed of a circuit chip and a chip antenna. The abovementioned air coils correspond to the chip antenna. While some circuit chips of the RFID chips have a size of approximately 1 x 1 mm, chip antennas connected to those circuit chips practically need to have a size of approximately several centimeters. It is difficult to dispose such a chip antenna on the cylinder flange of the chemical solution syringe.

In the chemical solution injector, the cylinder member of the chemical solution syringe is held at the cylinder flange and the piston member is slid into the held cylinder member, so that excessive stress is applied to the surface of the cylinder flange. The RFID chip and the air coils placed on the surface of the cylinder flange can break the RFID chip or the air coils. It is contemplated that the RFID chip and the air coils are placed within the cylinder flange to prevent such breaks, but that arrangement significantly reduces the productivity of the chemical solution syringe and thus is not a practical idea.

Then, the present applicant has considered the placement of an RFID chip on the outer circumference surface of a cylinder member of a chemical solution syringe and prototyped an RFID chip to test communication performance. The RFID chip used was a µ-chip (registered trademark) with a size of 10 x 60 mm for wireless communication at 2.45 GHz. Because of that elongated shape, the RFID chip is easily put on the cylinder member near the rear end such that it is wound thereon.

It has been revealed, however, that the abovementioned RFID chip did not provide sufficient output in wireless communication and could not achieve favorable wireless communication, unless an RFID reader is placed close thereto. Particularly, since the abovementioned RFID chip needs to be flat in use, the communication performance thereof is impaired when it is wound and thus bent on the outer circumference surface of the cylinder member.

To prevent this, it is contemplated that the RFID chip is put on the outer circumference surface of the cylinder member such that the longitudinal direction of the elongated RFID chip is in parallel with the longitudinal direction of the cylinder member. This causes the RFID chip to be placed adjacent to the chemical solution contained in the cylinder member. Since the communication performance of the RFID chip is impaired by liquid, the RFID chip placed as described above cannot perform favorable wireless communication with the RFID reader.

In addition, in the chemical solution injector including the abovementioned chemical solution syringe mounted thereon, the placement of the RFID reader near the rear end of the cylinder member is not easy structurally. It is difficult that the RFID chip wound near the rear end of the cylinder member performs favorable wireless communication with the RFID reader placed on the chemical solution injector.

### Disclosure of the Invention

The present invention has been made in view of the abovementioned problems, and it is an object thereof to provide a chemical solution injection system which allows favorable wireless communication between an RFID chip on a chemical solution syringe and an RFID reader on a chemical solution injector.

The chemical solution injection system according to the present invention includes a chemical solution syringe and a chemical solution injector. The chemical solution syringe includes a cylinder member, a piston member, an RFID chip, and an amplification antenna. The chemical solution injector includes a cylinder holding mechanism, a piston driving mechanism, and an RFID reader.

The cylinder member of the chemical solution syringe is formed in a cylindrical shape and includes a tubular conduit portion formed at a front end and an annular cylinder flange formed at the outer circumference of a rear end. The piston member is formed in a columnar shape and slidably inserted into the cylinder member from the back thereof. The cylinder holding mechanism of the chemical solution injector holds the cylinder member of the chemical solution syringe mounted interchangeably. The piston driving mechanism at least presses the piston member into the held cylinder member to inject a chemical solution into a patient.

The RFID chip of the chemical solution syringe is formed in a predetermined elongated shape and wound on the outer circumference surface of the cylinder member. The RFID reader performs wireless communication with the RFID chip on the held cylinder member. Since the amplification antenna of the chemical solution syringe is formed in a shape encircling the outer circumference surface of the cylinder member, and is placed in front of and in parallel with the RFID chip, the amplification antenna improves the communication performance of the RFID chip, reduced since it is bent.

Various means referred to in the present invention may be arranged to perform their functions, and may comprise dedicated hardware for performing a predetermined function, a data processing apparatus whose predetermined function is given by a computer program, a predetermined function performed in a data processing apparatus according to a computer program, or a combination thereof.

Various components referred to in the present invention do not need to be a separate entity. A plurality of components may be constructed as one member, a single component may be constructed by a plurality of members, a certain component may be part of another component, or a certain component may have a portion overlapping a portion of another component. Although forward and rearward directions are specified in the description of the present invention, these directions are defined for convenience to simply describe the relative relationship between components of the present invention and the definition does not limit any direction in manufacture or actual use when the present invention is implemented.

### Effect of the Invention

In the chemical solution injection system of the present invention, the RFID chip having the predetermined elongated shape is wound on the outer circumference surface of the cylinder member of the chemical solution syringe, and the amplification antenna having the shape encircling the outer circumference surface of the cylinder member is placed in front of and in parallel with the RFID chip. Thus, the amplification antenna improves the communication performance of the RFID chip reduced, since it is bent. As a result, the RFID chip of the chemical solution syringe can perform favorable wireless communication with the RFID reader of the chemical solution injector.

### Brief Description of the Drawings

Fig. 1 is a perspective view showing how to mount a chemical solution syringe on an injection head of a chemical solution injector according to an embodiment of the present invention;
Fig. 2 is a perspective view showing the outer appearance of the chemical solution injector;
Fig. 3 is a perspective view showing the outer appearance of a CT scanner serving as an imaging diagnostic apparatus;
Fig. 4 is a block diagram showing the circuit structure of a chemical solution injection system;
Fig. 5 is a perspective view showing the outer appearance of the chemical solution syringe;
Fig. 6 is a perspective view showing the outer appearance of an RFID chip;
Fig. 7 is a schematic block diagram showing the logical structure of the chemical solution injector;
Fig. 8 is a flow chart showing the first half of processing operation in the chemical solution injector;
Fig. 9 is a flow chart showing the latter half;
Fig. 10 is a flow chart showing processing operation in the CT scanner; and
Fig. 11 is a perspective view showing the outer appearances of injection heads of variations.

### Description of Reference Numerals

- 100: CHEMICAL SOLUTION INJECTOR
- 116: PISTON DRIVING MECHANISM
- 120: CYLINDER HOLDING MECHANISM
- 130: RFID READER
- 140: COMPUTER UNIT SERVING AS VARIOUS MEANS
- 150: OPERATION CONTROL MEANS
- 151: CHECK STORING MEANS
- 152: DATA COMPARING MEANS
- 153: ALARM OUTPUTTING MEANS
- 154: DATA ACCUMULATING MEANS
- 156: DATA HOLDING MEANS
- 157: DISPLAY CONTROL MEANS
- 158: INJECTION CONTROL MEANS
- 200: CHEMICAL SOLUTION SYRINGE
- 210: CYLINDER MEMBER
- 212: CONDUIT PORTION
- 213: CYLINDER FLANGE
- 220: PISTON MEMBER
- 230: RFID CHIP
- 240: AMPLIFICATION ANTENNA
- 300: CT SCANNER SERVING AS IMAGING DIAGNOSTIC APPARATUS
- 1000: CHEMICAL SOLUTION INJECTION SYSTEM

### Best Mode for Carrying Out the Invention

### [Configuration of Embodiment]

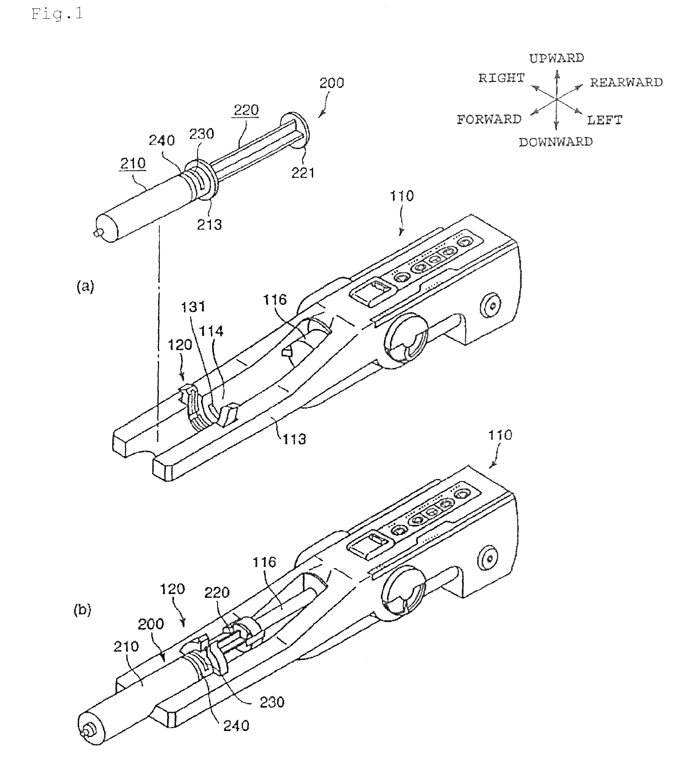

An embodiment of the present invention will hereinafter be described with reference to Figs. 1 to 11. As shown in Figs. 1 to 4, chemical solution injection system 1000 of the embodiment according to the present invention comprises chemical solution injector 100, chemical solution syringe 200, and CT scanner 300 which is an imaging diagnostic apparatus. The system is provided for taking diagnostic images of a patient (not shown) injected with a chemical solution such as a contrast medium, described later in detail.

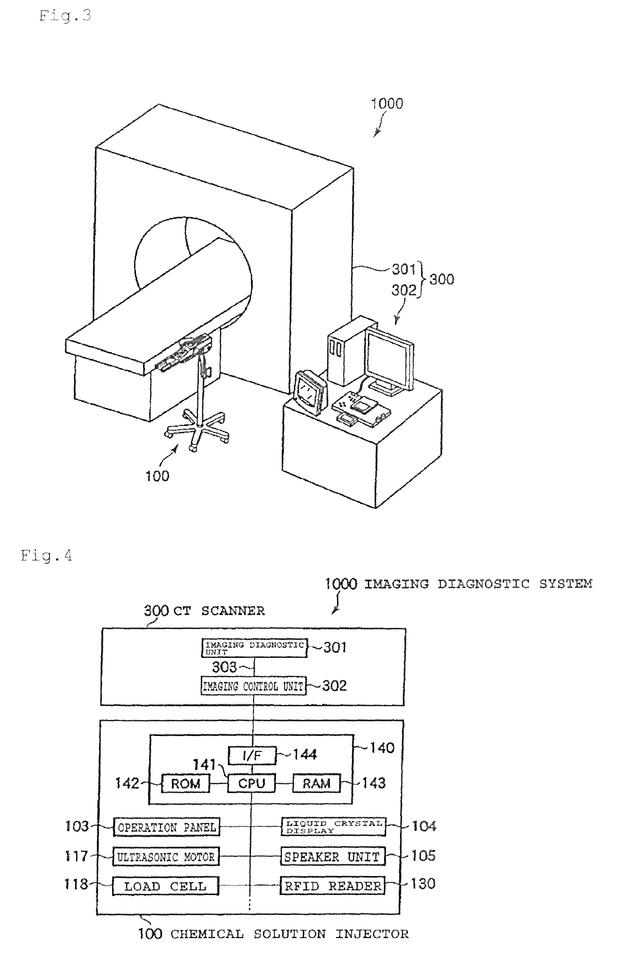

As shown in Figs. 3 and 4,CT scanner 300 includes imaging diagnostic unit 301 and imaging control unit 302. The imaging diagnostic unit 301 and imaging control unit 302 are wire-connected each other through communication network 303. Imaging diagnostic unit 301 shoots a diagnostic image of a patient. Imaging control unit 302 controls the operation of imaging diagnostic unit 301.

As shown in Figs. 1 and 5, chemical solution syringe 200 comprises cylinder member 210 and piston member 220 wherein piston member 220 is slidably inserted into cylinder member 210. Cylinder member 210 includes cylindrical hollow body 211, which has integrally formed conduit 212 in a tubular shape at the closed front end.

The rear end surface of body 211 of cylinder member 210 is opened, and piston member 220 is inserted from the opening into the interior of body 211. Cylinder member 210 has cylinder flange 213 formed on the outer circumference of the rear end, and piston member 220 has piston flange 221 formed on the outer circumference of the rear end.

In chemical solution injection system 1000 of the embodiment, at least some of chemical solution syringes 200 to be used are of the pre-filled type. Chemical solution syringe 200 of the pre-filled type is shipped with cylinder member 210 filled with a chemical solution. A RFID chip 230 is put on cylinder member 210 of chemical solution syringe 200. RFID chip 230 contains various types of data about chemical solution syringe 200 recorded thereon, such as the name, the identification data indicating the pre-filled type or the refill type, the identification data for each item, the capacity, the withstanding pressure of cylinder member 210, the inner diameter of cylinder member 210, and the stroke of piston member 220.

When chemical solution syringe 200 is pre-filled type, RFID chip 230 further contains various types of data about the contained solution set thereon, such as the name, the ingredients, the viscosity, the expiration date, and the identification data indicating whether the solution is for CT or for MR. When the solution in the syringe 200 is contrast medium, RFID chip 230 further contains data set thereon, as required, such as the operation pattern, with which the injection speed is changed over time.

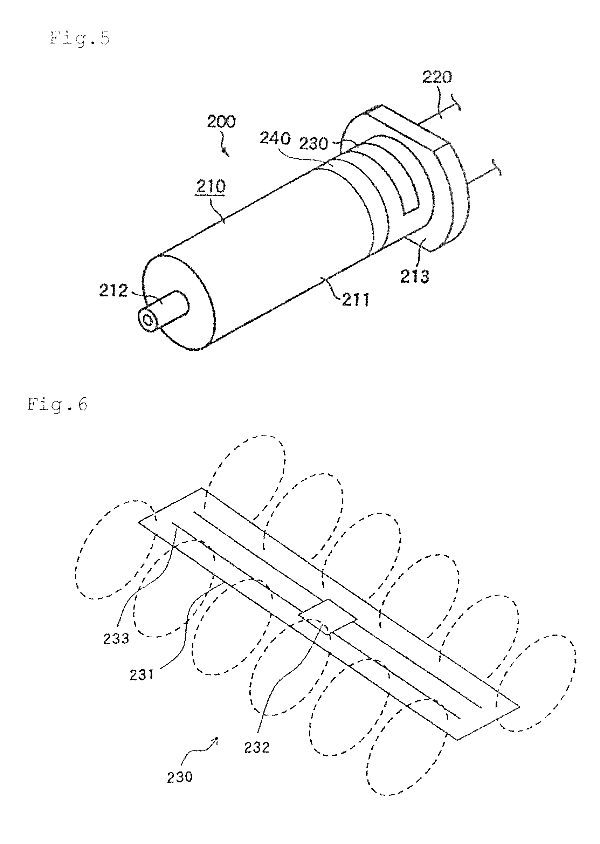

As shown in Fig. 6, RFID chip 230 has chip body 231 formed of elongated resin sheet. Circuit chip 232 is inserted substantially at the center of chip body 231. Chip antenna 233, made of linear antenna in a given shape, is also formed on chip body 231. Circuit chip 232 and chip antenna 233 are connected each other. For example, RFID chip 230 is realized by a µ-chip (registered trademark) in an elongated shape with a size of 10 x 60 (mm) for wireless communication at 2.45 (GHz).

As shown in Figs. 1 and 5, in chemical solution syringe 200 of the embodiment, RFID chip 230 in the elongated shape as described above is wound and put on the outer circumference surface of cylinder member 210, close to the rear end thereof. Amplification antenna 240 having a shape encircling the outer circumference surface of cylinder member 210 is put in front of RFID chip 230 in parallel therewith.

Amplification antenna 240 is formed of elongated copper foil having a width of approximately 5 mm, for example. Amplification antenna 240 is spaced from RFID chip 230 with a distance between the centers thereof of approximately 15 mm, which is generally an integral multiple of the communication wavelength at 2.45 GHz. At least when chemical solution syringe 200 is mounted on chemical solution injector 100, the front end of piston member 220 is positioned near the rear end of cylinder member 210. RFID chip 230 and amplification antenna 240 are overlapping with the front end of the piston member 220.

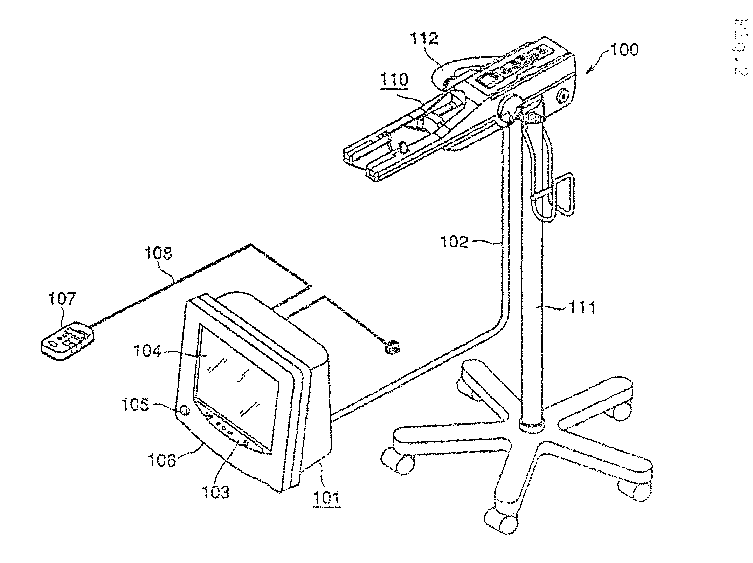

As shown in Fig. 2, chemical solution injector 100 of the embodiment has injection control unit 101 and injection head 110, constructed as separate components, which are wire-connected through communication cable 102. Injection head 110 drives chemical solution syringe 200 mounted thereon, to inject a chemical solution therefrom into a patient. Injection control unit 101 controls the operation of injection head 110.

Injection head 110 is attached to the top end of caster stand 111 by movable arm 112. As shown in Fig. 1, head body 113 of injection head 110 has concave portion 114 formed in the upper surface as a semi-cylindrical groove for removably mounting the syringe 200. Cylinder holding mechanism 120 is formed in the forward section of concave portion 114 for removably holding cylinder flange 211 of chemical solution syringe 200. Piston driving mechanism 116 is placed in the rearward section of concave portion 114 for holding and sliding piston flange 221.

As shown in Fig. 4, piston holding mechanism 116 has ultrasonic motor 117 as a driving source which is free from generation of magnetic field even in operation, and slides piston member 220 through a screw mechanism (not shown) or the like. Load cell 118 is also contained in piston driving mechanism 116 and detects the pressure applied to piston member 220.

As shown in Fig. 4, chemical solution injector 100 of the embodiment comprises a RFID reader 130, which wirelessly communicates with the RFID chip 230 of the syringe 200. RFID reader 130 has a communication circuit (not shown) and reader antenna 131. The communication circuit is contained, for example, in the rearward section of injection head 110.

As shown in Fig. 1, reader antenna 131 is formed of elongated conductor sheet, serving as linear antenna, and is connected to the communication circuit. Reader antenna 131 is put on the bottom of concave portion 114, at the rear of cylinder holding mechanism 120. The antenna 131 extends along left-to-right direction. Reader antenna 131 is put on a plane generally in parallel with the circular plane formed by amplification antenna 240 on held chemical solution syringe 200.

As shown in Fig. 4, injection control unit 101 connected to injection head 110 formed as described above through communication cable 102, contains a computer unit 140, and is connected to imaging control unit 302 of CT scanner 300 through communication network 304.

As shown in Fig. 2, injection control unit 101 has operation panel 103, chemical solution crystal display 104 serving as a display means, and speaker unit 105, all of which are disposed on the front face of unit housing 106. Injection control unit 101 is wire-connected to controller unit 107 as a separate component through connector 108.

As shown in Fig. 4, in chemical solution injector 100 of the embodiment, the abovementioned various devices are connected to computer unit 140 which integrates and controls those various devices. Computer unit 140 is formed of a so-called one-chip microcomputer provided with hardware such as CPU (Central Processing Unit) 141, ROM (Read Only Memory) 142, RAM (Random Access Memory) 143, I/F (Interface) 144 and the like. Computer unit 140 has an appropriate computer program installed as firmware or the like in an information storage medium such as ROM 142, and CPU 141 executes various types of processing in accordance with the computer program.

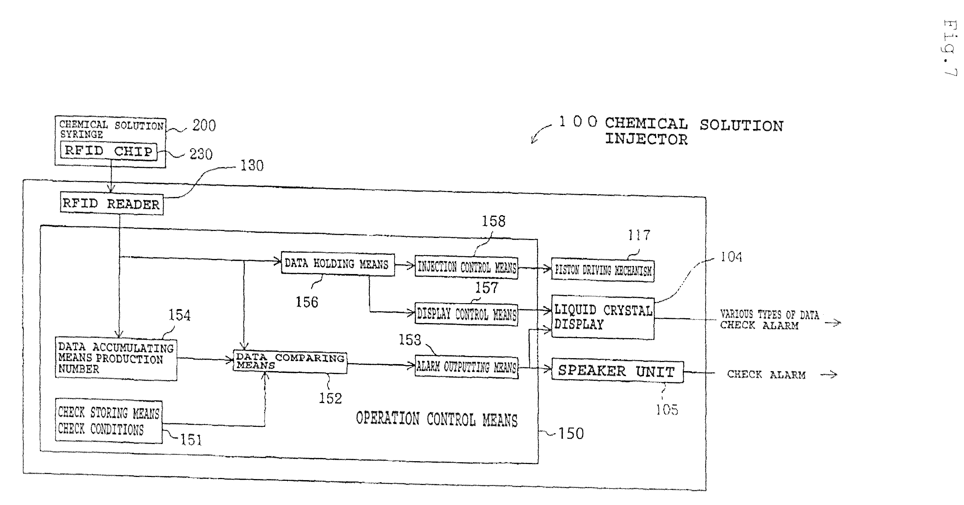

In chemical solution injector 100 of the embodiment, computer unit 140 operates in accordance with the computer program installed as described above to logically have operation control means 150 as shown in Fig. 7. Operation control means 150 logically has various means such as check storing means 151, data comparing means 152, alarm outputting means 153, data accumulating means 154, data holding means 156, display control means 157, and injection control means 158.

Operation control means 150 corresponds to the function of CPU 141 which performs predetermined operations in accordance with the computer program installed in ROM 142 or the like and the various types of data wirelessly received from RFID chip 230.

Check storing means 151 corresponds to the store area of RAM 143 and the like recognized by CPU 141, and stores predetermined check conditions as data. Data comparing means 152 compares the check conditions stored as data with the various types of data wirelessly received from RFID chip 230. Alarm outputting means 153 outputs and notifies a check alarm in accordance with the comparison result.

More particularly, RAM 143 stores data as check condition, for identifying usable chemical solution syringe 200 registered thereon. When RFID reader 130 wirelessly receives various types of data from RFID chip 230 of chemical solution syringe 200, the wirelessly received identification data of chemical solution syringe 200 is compared with the identification data stored in RAM 143.

When the wirelessly received identification data does not match the stored data, a guidance message, for example "This product not registered as usable device. Check if it is usable" is output as a check alarm on liquid crystal display 104, with sound from speaker unit 105.

The current date and time is constantly updated and held in the check conditions on RAM 143. When the expiration date is wirelessly received from RFID chip 230 of chemical solution syringe 200, the expiration date is compared with the current date and time. If the current data and time is after the expiration date, a guidance message, for example "Expiration date of this product elapsed. Use new one" is output as a check alarm on liquid crystal display 104, with sound from speaker unit 105.

Because the production number of each chemical solution syringe 200 of the pre-filled type is stored on RFID chip 230, data accumulating means 154 stores the data of the production number of chemical solution syringe 200 of the pre-filled type put on injection head 110 and used to perform injection operation.

Data comparing means 152 compares the stored production number with the production number wirelessly received from RFID chip 230. When the numbers match, alarm outputting means 153 outputs a guidance message, for example "This pre-filled syringe used previously. Use new one" as a check alarm on liquid crystal display 104 and with sound from speaker unit 105.

Data holding means 156 holds various types of data wirelessly received from RFID chip 230. Display control means 157 displays the held various types of data on liquid crystal display 104. Injection control means 158 controls the operation of piston driving mechanism 116 based on the held various types of data.

More specifically, RFID chip 230 of chemical solution syringe 200 contains various types of data, such as the name, the withstanding pressure, and the capacity of chemical solution syringe 200, as well as the name, the ingredients, and the expiration date of the chemical solution in the syringe 200. The various types of data are temporarily stored in RAM 143 and output on liquid crystal display 104.

When the control data for piston driving mechanism 116 is set on RFID chip 230 of chemical solution syringe 200, the control data is held in RAM 143 and CPU 141 controls the operation of piston driving mechanism 116 based on the held control data.

For example, when an operation pattern for changing the injection speed of the contrast medium over time is recorded in RFID chip 230 of chemical solution syringe 200, CPU 141 changes the operation speed of piston driving mechanism 116 over time in accordance with the operation pattern. When the withstanding pressure is recorded on RFID chip 230 of chemical solution syringe 200, CPU 141 controls the operation of piston driving mechanism 116, based on the pressure detected by load cell 118, such that the withstanding pressure stored in RAM 143 is not exceeded. When the capacity is recorded on RFID chip 230 of chemical solution syringe 200, CPU 141 controls the operation of piston driving mechanism 116, based on the capacity held as data on RAM 143.

Although the abovementioned various means of chemical solution injector 100 are accomplished by pieces of hardware such as liquid crystal display 104 as required, they are mainly implemented by CPU 141 as a piece of hardware functioning in accordance with the resources and the computer program stored on an information storage medium such as ROM 142.

Such a computer program is stored in an information storage medium such as RAM 143, as software for causing CPU 141 or the like, to perform processing operations including following processes:
comparing the check conditions stored in RAM 143 and the like with the various types of data wirelessly received from RFID chip 230 ,when RFID reader 130 receives the various types of data from RFID chip 230,
outputting check alarm on liquid crystal display 104, in accordance with the comparison result,
storing production number of chemical solution syringe 200 mounted and used to perform injection operation in RAM 143 or the like,
comparing a stored production number with a production number, wirelessly received as data from RFID chip 230,
outputting check alarm on liquid crystal display 104 in accordance with the comparison result,
storing the various types of data wirelessly received from RFID chip 230 on RAM 143 or the like,
outputting the various types of data on liquid crystal display 104,
controlling an operation of piston driving mechanism 116, in accordance with the various types of data.

### [Operation of the Embodiment]

When chemical solution injection system 1000 of the embodiment is used in the abovementioned structure, injection head 110 of chemical solution injector 100 is placed near imaging unit 301 of CT scanner 300, and chemical solution syringe 200 or the like is prepared for use as shown in Fig. 3. An operator opens flange holding mechanism 120 of injection head 110 and puts chemical solution syringe 200 in concave portion 114 to insert cylinder flange 213 into flange holding mechanism 120 and then closes flange holding mechanism 120.

When chemical solution syringe 200 is mounted on injection head 110 in this manner, reader antenna 131 of injection head 110 is positioned on the plane generally in parallel with the plane formed by amplification antenna 240 adjacent to RFID chip 230 of chemical solution syringe 200. Then, RFID chip 230 wirelessly communicates with RFID reader 130.

The underlying principles will be described in brief. RFID chip 230 wirelessly communicates with RFID reader 130 through an electric field (radio communication) and a magnetic field (magnetic coupling), now attention is focused on the magnetic field to simplify the description. As shown in Fig. 6, chip antenna 233 of RFID chip 230 is formed in an elongated linear shape, so that the magnetic field is produced cylindrically with its longitudinal direction as the axis.

Since reader antenna 131 of RFID reader 130 is also formed in an elongated linear shape, the magnetic field is produced cylindrically with its longitudinal direction as the axis. Chip antenna 233 and reader antenna 131 are favorably coupled magnetically when they are placed in parallel. But when they are not placed in parallel, they are not satisfactorily coupled magnetically. When RFID chip 230 is bent, the shape of its magnetic field is also bent, which impairs the magnetic coupling between chip antenna 233 and reader antenna 131.

In chemical solution injection system 1000 of the embodiment, however, circular amplification antenna 240 is adjacent to RFID chip 230 bent in an arc shape, and reader antenna 131 is placed on the plane generally in parallel with the plane formed by amplification antenna 240. Thus, the magnetic field of RFID chip 230 is amplified by the magnetic coupling to amplification antenna 240, and the magnetic field is isotropic in the direction of the plane formed by circular amplification antenna 240. As a result, chip antenna 233 and reader antenna 131 are favorably coupled magnetically to achieve wireless communication between RFID chip 230 and RFID reader 130.

The present applicant has prototyped chemical solution syringes including amplification antennas with various shapes and arrangements to test communication performance between RFID chip 230 and RFID reader 130. First, when no amplification antenna was provided, in chemical solution syringe 200 of a large diameter on which RFID chip 230 was less bent, wires communication was possible by rotating chemical solution syringe 200 about its axis to place RFID chip 230 generally in parallel with reader antenna 131. However, if they were not generally in parallel, wireless communication was not possible. In this manner, it was recognized that RFID chip 230 and reader antenna 131 had high directivity.

In chemical solution syringe 200 of a small diameter on which RFID chip 230 was extremely bent, wireless communication was not possible between RFID chip 230 and RFID reader 130 regardless of the rotation direction of chemical solution syringe 200. It was recognized that the communication performance of RFID chip 230 was reduced when it was bent.

A short and small amplification antenna was placed at the rear of RFID chip 230 in parallel therewith. It was found that the amplification antenna reduced the communication performance. In addition, an amplification antenna, which is longer than RFID chip 230 but did not encircle cylinder member 210, was put in front of RFID chip 230 in parallel therewith. It was recognized that the communication performance was improved only slightly.

When amplification antenna 240 having a shape encircling cylinder member 210 was placed in front of RFID chip 230 in parallel, it was found that the communication performance was drastically improved, and that favorable wireless communication was possible even when RFID chip 230 is not generally in parallel with reader antenna 131. It is contemplated that this is because the flow of induced current around circular amplification antenna 240 satisfactorily amplified the magnetic field of RFID chip 230 and made the magnetic field isotropic.

The present inventor changed the distance between the centers of amplification antenna 240 encircling cylinder member 210 and RFID chip 230, and found that the best communication performance was achieved when the distance between the centers was equal to the communication wavelength of RFID reader 130 and RFID chip 230. It is contemplated that this is because the distance between the centers of amplification antenna 240 and RFID chip 230 set to be equal to the communication wavelength caused favorable resonance in RFID chip 230 and amplification antenna 240.

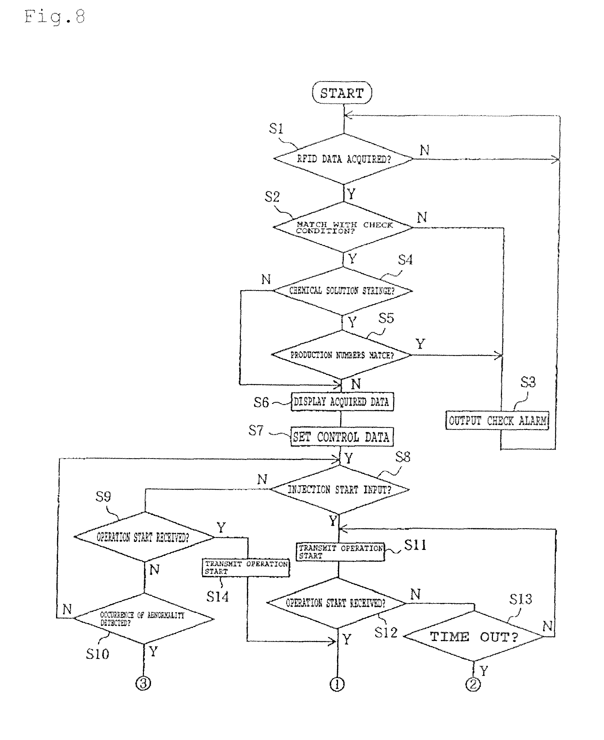

In chemical solution injection system 1000 of the embodiment, when chemical solution syringe 200 is mounted on chemical solution injector 100 as described above, RFID chip 230 and RFID reader 130 performs favorable wireless communication regardless of the rotation direction of chemical solution syringe 200. Then, as shown in Fig. 8, computer unit 140 compares various types of data wirelessly received from RFID chip 230 by RFID reader 130 (step S1) with the check conditions registered on RAM 143 (step S2).

Such check conditions include the identification data of usable chemical solution syringe 200. If the identification data wirelessly received from RFID chip 230 is not included in the check conditions, a guidance message, for example "This product not registered as usable device. Check if is usable" is output as a check alarm with display on liquid crystal display 104 and with sound from speaker unit 105 (step S3).

When chemical solution syringe 200 is appropriately mounted on injection head 110, RFID chip 230 thereon is faced toward RFID reader 130 with a predetermined interval between them. Various types of data on RFID chips 230 are wirelessly received by RFID reader 130 (step S1).

The wirelessly received data is compared with the check conditions (step S2), and if the wirelessly received identification data is not included in the check conditions, a check alarm is output (step S3). Even after the data matches the check conditions, when it is determined that the device to be used is chemical solution syringe 200 (step S4), the production number wirelessly received from RFID chip 230 is compared with the production number registered in RAM 143 (step S5).

When the compared production numbers match each other, a guidance message, for example "This syringe used previously. Use new one" is output as a check alarm on liquid crystal display 104 and from speaker unit 105 (step S3).

Incidentally the various types of data wirelessly received from RFID chip 230 on the appropriate device into chemical solution injector 100 as described above are output with display on chemical solution crystal display 104, for example as "Contrast medium syringe (name) made by (manufacturer) mounted. Production number XXX, name of chemical solution XXX, type of chemical solution XXX, capacity XXX, withstanding pressure XXX" (step S6).

RFID chip 230 has various types of data to be displayed and various types of data not to be displayed. For example, a binary flag is set in each data to indicate whether or not the data should be displayed. Chemical solution injector 100 appropriately selects some of the various types of data wirelessly received from RFID chip 230 for display.

When the various types of data wirelessly received from RFID chip 230 on the device into chemical solution injector 100 include control data such as "withstanding pressure," "capacity," and "operation pattern for changing the injection speed of the contrast medium over time," , then the control data is set in RAM 143 of computer unit 140 (step S7). When such control data is not included in the data wirelessly received from RFID chip 230, default control data is set.

As described above, chemical solution syringe 200 mounted on chemical solution injector 100 is connected to a patient through an extension tube (not shown) or the like and then the operator makes entry to start operation to operation panel 103. Then, chemical solution injector 100 detects the entry (step S8), and transmits data for starting operation to CT scanner 300 (step S11).

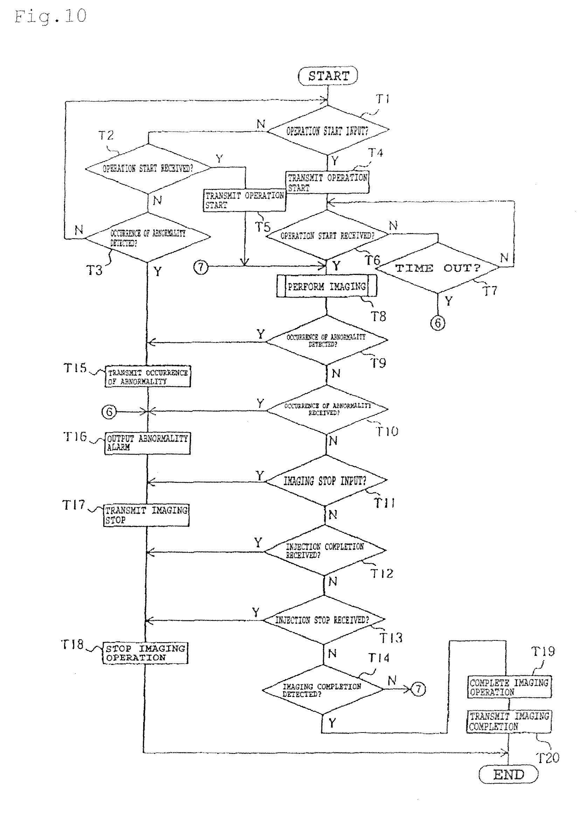

Referring to Fig. 10, CT scanner 300 receives the data for staring operation from chemical solution injector 100 (step T2) and transmits the data for starting operation back to chemical solution injector 100 and performs imaging operation (step T8). Thus, in imaging diagnostic system 1000 of the embodiment, the imaging of CT scanner 300 follows the injection of chemical solution injector 100.

As shown in Figs. 8 and 10, in imaging diagnostic system 1000 of the embodiment, when chemical solution injector 100 is ready as described above (steps S8 to S10) and the operator makes entry to start operation to CT scanner 300 (step T1), the chemical solution injection of chemical solution injector 100 follows the imaging of CT scanner 300 (steps T4, T6 and subsequent steps, steps S9, S18 and subsequent steps).

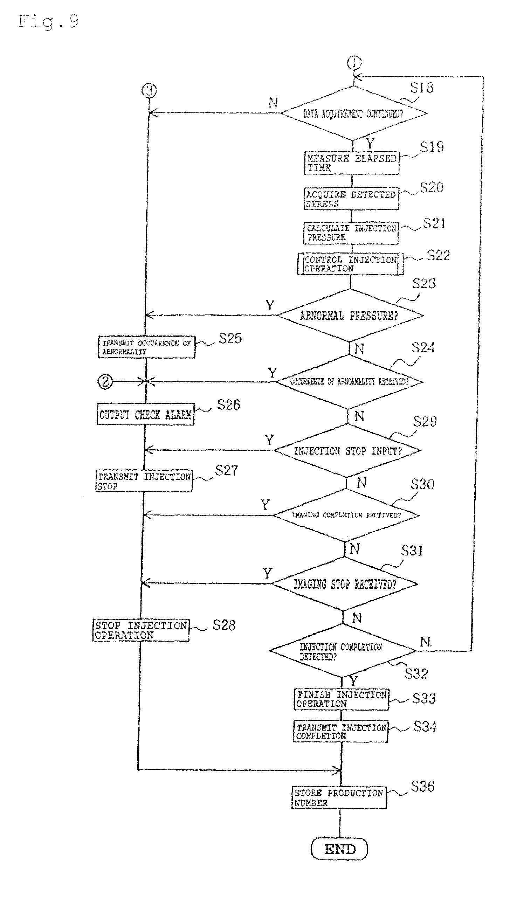

As shown in Fig. 9, when a series of chemical solution injection operations is performed (step S18 and subsequent steps) in chemical solution injector 100 of the embodiment, the elapsed time from the start of the injection is counted (step S19), and the operation of piston driving mechanism 116 is controlled in real time, based on the elapsed time and the control data, wirelessly received from RFID chip 230 (step S22).

If the operation pattern for changing the injection speed of the contrast medium over time is set in RFID chip 230 of chemical solution syringe 200, the operation speed of piston driving mechanism 116 is changed over time in accordance with the operation pattern. When piston driving mechanism 116 is driven as described above, the stress detected by load cell 118 is wirelessly received in real time by computer unit 140 (step S20).

The injection pressure of the chemical solution is calculated from the stress detected by load cell 118 (step S21) based on the viscosity of the chemical solution, the inner diameter of cylinder member 210 and the like wirelessly received from RFID chip 230. The operation of piston driving mechanism 116 is controlled in real time such that the injection pressure satisfies the pressure range, wirelessly received from RFID chip 230 (step S23). When the withstanding pressure is set on RFID chip 230 of chemical solution syringe 200, the operation of piston driving mechanism 116 is controlled in accordance with the withstanding pressure.

While chemical solution syringe 200 is driven by piston driving mechanism 116 as described above, RFID chip 230 is continuously detected by RFID reader 130 (step S18). If the abovementioned detection is stopped before the completion of the injection operation (step S32), the injection operation performed by piston driving mechanism 116 is stopped (step S28).

In addition, a guidance message, for example "Syringe removal detected. Make sure syringe put appropriately" is output as a check alarm with display on liquid crystal display 104 and with sound from speaker unit 105 (step S26). The occurrence of abnormality and the stop of injection are transmitted as data to CT scanner 300 (steps S25 and S28).

Then, CT scanner 300 receives the data representing the occurrence of abnormality (step T10) and outputs the occurrence of abnormality as a check alarm with guidance display or the like (step T16). When it receives the data representing the stop of operation (step T13), the imaging operation is stopped (step S18).

In chemical solution injector 100 and CT scanner 300 of the embodiment, when the occurrence of abnormality is detected in the abovementioned ready state (steps S10 and T3) or when the occurrence of abnormality is detected during the operation (steps S23 and T9), the occurrence of abnormality is output and notified (steps S26 and T16) and the operation is stopped (steps S28 and T18).

Since the occurrence of abnormality in one of them is transmitted to the other (steps S25 and T15), the other receives the data (steps T10 and S24) and then outputs and notifies the occurrence of abnormality (steps T16 and S26). Since the operation stop in one of them is transmitted to the other (steps S27 and T17), the other receives the data (steps T13 and S31) and stops the operation (steps T18 and S28).

When one of them receives entry to stop operation (steps S29 and T11), the one stops the operation (steps S28 and T18) and transmits it to the other (steps S27 and T17). The other receives the data (steps T13 and S31) and stops the operation (steps T18 and S28).

When the completion of the operation is detected in one of them (steps S32 and T14), the operation is ended (steps S33 and T19) and the end of the operation is transmitted to the other (steps S34 and T20). The other receives the data (steps T12 and S31) and stops the operation (steps T18 and S28).

In chemical solution injector 100 of the embodiment, when the injection operation is finished normally or abnormally as described above (steps S33 and S28), the identification data wirelessly received from RFID chip 230 of chemical solution syringe 200 is registered as the check condition in RAM 143 (step S36).

### [Effect of the Embodiment]

In chemical solution injection system 1000 of the embodiment, RFID chip 230 contains the various types of data recorded thereon is put on chemical solution syringe 200 as described above. Chemical solution injector 100 wirelessly receives the various types of data from RFID chip 230 and performs the predetermined operation in accordance with at least some of the various types of data. In this manner, a large amount of data can be easily entered into chemical solution injector 100 to perform various operations.

When RFID chip 230 is an off-the-shelf product, for example, formed in an elongated shape measuring 10 x 60 mm, the chip 230 cannot be put on cylinder flange 213 of chemical solution syringe 200. In case RFID chip 230 is put on the outer surface of the cylinder member 210, extending along its longitudinal direction, the performance of communication between RFID chip 230 and antenna 131 is impaired by the effect of solution.

Thus, in practical ways, the RFID chip 230 with elongated shape is preferable to be wound on the outer circumference surface of cylinder member 210. However, the communication performance of RFID chip 230 deteriorates if RFID chip 230 is put in an arc shape. Incidentally, the reader antenna 131 and chip antenna 233 formed of linear antennas of RFID reader 130 and RFID chip 230, respectively, have high directivity, thus they do not perform favorable wireless communication unless they are placed in parallel.

In chemical solution injection system 1000 of the embodiment, elongated RFID chip 230 is wound on the outer circumference surface of cylinder member 210 of chemical solution syringe 200, and amplification antenna 240 of the shape encircling the outer circumference surface of cylinder member 210 is placed in front of RFID chip 230 in parallel.

With that arrangement, the communication performance of RFID chip 230, which is reduced due to the bending in the arc shape, is enhanced by amplification antenna 240, thereby achieving favorable wireless communication between RFID reader 130 and RFID chip 230. In addition, since amplification antenna 240 in the circular shape generally eliminates the directivity of reader antenna 131 and chip antenna 233, RFID reader 130 and RFID chip 230 can wirelessly communicate with each other regardless of the rotation direction of chemical solution syringe 200. Especially, since the distance between the centers of RFID chip 230 and amplification antenna 240 is comparable to the wavelength in the wireless communication, the communication performance of RFID chip 230 can be satisfactorily improved by amplification antenna 240.

In chemical solution injection system 1000 of the embodiment, reader antenna 131 of RFID reader 130 is formed of the linear antenna having high directivity. Reader antenna 131 is placed on the plane, generally in parallel with the plane formed by amplification antenna 240, thus the wireless communication between RFID reader 130 and RFID chip 230 via amplification antenna 240 can be performed well.

The communication performance of RFID chip 230 is impaired by liquid as described above. In chemical solution syringe 200 of the embodiment, however, the front end of piston member 220 is placed at the rear end of cylinder member 210, and RFID chip 230 and amplification antenna 240 are placed at the positions overlapping the front end of piston member 220. As a result, RFID reader 130 and RFID chip 230 can favorably communicate wirelessly with each other via amplification antenna 240.

Particularly, RFID reader 130 and RFID chip 230 perform wireless communication at the frequency of 2.45 GHz, and the distance between the centers of RFID chip 230 and amplification antenna 240 can be set to approximately 15 mm. RFID chip 230 and amplification antenna 240 are placed at the positions overlapping piston member 220 of cylinder member 210 without any problem.

In chemical solution injection system 1000 of the embodiment, computer unit 140 allows piston driving mechanism 116 to operate only when RFID reader 130 detects RFID chip 230. If chemical solution syringe 200 comes off injection head 110 during injection, the chemical solution injection operation can be stopped automatically.

Since the mechanism for detecting the appropriate mounting of chemical solution syringe 200 is formed of RFID chip 230 and RFID reader 130 for transmitting the various types of data from chemical solution syringe 200 to chemical solution injector 100, the appropriate mounting of chemical solution syringe 200 can be detected by using the simple structure without requiring a dedicated sensor mechanism.

In chemical solution injection system 1000 of the embodiment, at least some of the various types of data wirelessly received from RFID chip 230 are held as data and output with display on liquid crystal display 104. The operator can check the various types of data of chemical solution syringe 200 and the like easily and reliably.

Chemical solution injector 100 of the embodiment compares the check conditions stored as data with the various types of data wirelessly received from RFID chip 230, and as required, outputs the check alarm. For example, when the operator attempts to use chemical solution syringe 200 which is not allowed in chemical solution injector 100 or chemical solution syringe 200 with the expiration date elapsed, the check alarm can be output to prevent any medical malpractice reliably.

Particularly, in chemical solution injector 100 of the embodiment, when the data is read from RFID chip 230 of chemical solution syringe 200, the production number of each item is stored. If the production number newly received wirelessly from RFID chip 230 is already stored, the check alarm is output. It is thus possible to readily and reliably prevent medical malpractice such as repeated use of chemical solution syringe 200 which should be discarded once it is used.

In chemical solution injection system 1000 of the embodiment, when the operation pattern for changing the injection speed of the constant medium over time is recorded on RFID chip 230 of chemical solution syringe 200 of the pre-filled type filled with the contrast medium, chemical solution injector 100 changes the injection speed of the contrast medium over time in accordance with the operation pattern.

Consequently, the optimal image contrast can be maintained favorably, and the minimum amount of the injected contrast medium can be used to reduce physical burdens on the patient. In addition, it is not necessary to previously register the data of the complicated operation pattern in chemical solution injector 100. For example, a new operation pattern for a new contrast medium can be simply input as data to chemical solution injector 100 from RFID chip 230 of chemical solution syringe 200.

In chemical solution injector 100 of the embodiment, the pressure of the injected chemical solution is detected from the stress on piston member 220 of chemical solution syringe 200, and if the injection pressure reaches an abnormal value, the check alarm is output and the injection operation is forcedly stopped. This can prevent medical malpractice of injection of the chemical solution at an abnormal pressure.

The detection of the pressure of the chemical solution by chemical solution injector 100 as described above requires not only the stress on piston member 220 of chemical solution syringe 200 but also the various types of data such as the inner diameter of cylinder member 210 and the viscosity of the solution. The various types of data are input to chemical solution injector 100 from RFID chip 230. Thus, in chemical solution injection system 1000 of the embodiment, chemical solution injector 100 can appropriately detect the injection pressure of each chemical solution of chemical solution syringe 200 without requiring complicated operations of manual entry of the various types of data into chemical solution injector 100 by the operator.

In imaging diagnostic system 1000 of the embodiment, since the injection in chemical solution injector 100 is automatically associated with the imaging in CT scanner 300, the diagnostic images can be taken in an appropriate timing from the patient injected with the contrast medium in an appropriate timing.

### [Modifications of the Embodiment]

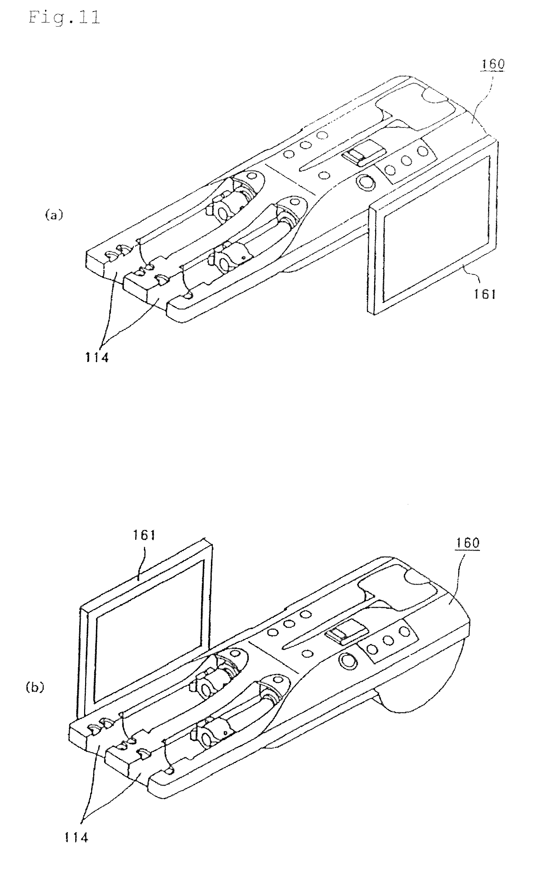

The present invention is not in any way limited to the abovementioned embodiment, but various changes and modifications may be made therein without departing from the scope of the invention. For example, in the above embodiment, only one chemical solution syringe 200 is mounted in one concave portion 114 of injection head 110 in chemical solution injector 100. As shown in Fig. 11, it is possible to provide a chemical solution injector (not shown) in which a plurality of chemical solution syringes 200 are individually mounted in a plurality of concave portions 114 of injection head 160.

In this case, RFID reader 130 can be provided for each concave portion 114 of injection head 160, and recorded data can be detected from each of RFID chips 230 of the plurality of chemical solution syringes 200. Since RFID readers 130 can detect data on the plurality of RFID chips 230 on a time-division basis, RFID reader 130 can include one communication circuit and a plurality of reader antennas 131 placed individually in the plurality of concave portions 114, for example.

In the above embodiment, the recorded data detected by RFID reader 130 from RFID chip 230 is output with display on liquid crystal display 104 of injection control unit 101 separate from injection head 110. However, as shown in Fig. 11, display panel 161 may be mounted on injection head 160 and recorded data from RFID chip 230 may be output on display panel 161.

In this case, immediately after chemical solution syringe 200 is appropriately mounted in injection head 160, recorded data is output with display on display panel 161 of injection head 160. This allows immediate check of appropriate mounting of chemical solution syringe 200 and intuitive recognition of the displayed data.

In the above embodiment, the distance between the centers of RFID chip 230 and amplification antenna 240 in chemical solution syringe 200 is generally the integral multiple of the communication wavelength. The distance between the centers may be 1/2ⁿth (n is a natural number) of the communication wavelength. Furthermore, the above embodiment has been described in conjunction with the product which performs wireless communication with microwaves at 2.45 GHz as RFID chip 230, but it is possible to use a product for wireless communication with UHF waves at 900 MHz or the like as RFID chip 230, for example (not shown).

In the above embodiment, reader antenna 131 of RFID reader 130 placed on the plane generally in parallel with the plane formed by amplification antenna 240 of chemical solution syringe 200 mounted on injection head 110 is formed of the linear antenna. For example, the reader antenna may be formed of a circular polarization antenna or a planar antenna (not shown).

Since piston member 220 and piston driving mechanism 16 are disposed at the back of cylinder member 210 of chemical solution syringe 200, it is not easy to place the circular polarization antenna or the planar antenna on the plane generally in parallel with the plane formed by amplification antenna 240. Thus, when the reader antenna is realized by a circular polarization antenna or a planar antenna, the reader antenna may be placed at a position shifted leftward, rightward, upward, or downward from the position of piston member 220 or piston driving mechanism 116, or the reader antenna may be incorporated in the forward surface of piston driving mechanism 116, for example.

In the above embodiment, to use chemical solution syringe 200 or the like only once, the data of the production number of each chemical solution syringe 200 is wirelessly received from RFID chip 230 of chemical solution syringe 200 by RFID reader 130 and stored in chemical solution injector 100, and if a newly wirelessly received production number is already stored, the check alarm is output.

Alternatively, it is possible that a rewritable product is used as RFID chip 230 of chemical solution syringe 200, chemical solution injector 100 records on RFID chip 230 of chemical solution syringe 200 the data "used" or the fact that chemical solution syringe 200 has been mounted and the solution thereof has been injected, and a check alarm is output when the data "used" is wirelessly received from RFID chip 230 of newly mounted chemical solution syringe 200.

In this case, multiple production numbers do not need to be stored in chemical solution injector 100, an overflow or the like of RAM 143 can be prevented, and RAM 143 having a large capacity does not need to be included uselessly. In addition, even when the data stored in chemical solution injector 100 is erroneously reset, inappropriately repeated use of chemical solution syringe 200 or the like can be prevented.

In the above embodiment, the control data or the like for the injection is wirelessly received from RFID chip 230 of chemical solution syringe 200 to chemical solution injector 100, and chemical solution injector 100 controls the operation of the injection based on the control data. It is also possible that chemical solution injector 100 controls the operation of the injection, based on a combination of control data wirelessly received from RFID chip 230 of chemical solution syringe 200 and control data entered through operation panel 103 or the like.

For example, it is possible that the operation pattern of liquid injection over time is recorded on RFID chip 230 of chemical solution syringe 200 as described above, and when an operator enters the data of an area to be imaged by CT scanner 300 through operation panel 103 or the like, the operation pattern is adjusted in accordance with the area to be imaged.

In the above embodiment, chemical solution injector 100 finishes the injection operation and registers the production number wirelessly received from RFID chip 230 of chemical solution syringe 200, and then ends the various types of operations. Alternatively, for example, it is possible that when chemical solution injector 100 finishes the injection operation and registration of the production number as described above and detects removal of chemical solution syringe 200 with RFID reader 130, chemical solution injector 100 automatically moves piston driving mechanism 116 backward to the initial position at the backend.

It is also possible that when chemical solution injector 100 completes the various types of operations and moves piston driving mechanism 116 back to the initial position and then detects the mounting of new chemical solution syringe 200 with RFID reader 130, chemical solution injector 100 automatically moves piston driving mechanism 116 forward to the standby position for holding piston members 210. In this case, chemical solution syringe 200 can be removed and put in chemical solution injector 100 in an appropriate timing to place piston driving mechanism 116 automatically to the appropriate position, so that any special operation is not required to place piston driving mechanism 116 and the convenience can be improved.

In the above embodiment, the various types of data are recorded by the manufacturer on RFID chip 230 of chemical solution syringe 200. Alternatively, the various types of data may be recorded on RFID chip 230 of chemical solution syringe 200 or the like in a medical facility such as a hospital where chemical solution syringe 200 is used.

In this case, desired data can be provided for chemical solution syringe 200 in the medial facility. For example when a desired solution is filled into chemical solution syringe 200 of the refill type, various types of data of the solution can be recorded on RFID chip 230. In such a case, however, it is preferable that the production number is previously recorded on RFID chip 230 to prevent repeated use of chemical solution syringe 200 as described above.

In the above embodiment, CT scanner 300 is used as the imaging diagnostic apparatus and chemical solution injector 100 injects the contrast medium for CT. For example, an MRI apparatus or a PET apparatus may be used as the imaging diagnostic apparatus and the chemical solution injector may inject a contrast medium therefor.

In the above embodiment, the respective portions of chemical solution injector 100 have been specifically described, but the portions may be changed in various manners. For example, the driving source of the piston driving mechanism may be realized by a DC (Direct Current) motor or an AC (Alternating Current) motor, or the display panel may be realized by an organic EL (Electro-Luminescence) display or a plasma display (not shown).

In the above embodiment, CPU 141 operates in accordance with the computer program stored in RAM 143 or the like to realize logically various means as various functions of chemical solution injector 100. Each of the various means may be formed as specific hardware, or some of them may be stored as software on ROM 143, while others may be formed as hardware.

### Industrial Availability

The present invention is used in a chemical solution injection system for injecting a contrast medium into a patient with an imaging diagnostic apparatus such as a CT (Computed Tomography) scanner, for example.

## Claims

1. A chemical solution injection system at least comprising:
a chemical solution syringe including a cylindrical cylinder member and a columnar piston member slidably inserted into the cylinder member from the back thereof, the cylinder member including a tubular conduit portion formed at a front end and an annular cylinder flange formed at a rear end on outer circumference; and
a chemical solution injector for injecting the chemical solution into a patient by relatively moving the cylinder member and the piston member of the chemical solution syringe mounted interchangeably,
wherein the chemical solution syringe includes
a RFID (Radio Frequency Identification) chip having a predetermined elongated shape, wound on the outer circumference surface of the cylinder member, and an amplification antenna having a shape encircling the outer circumference surface of the cylinder member, placed in front of and in parallel with the RFID chip,
the chemical solution injector includes
a cylinder holding mechanism for holding the cylinder member,
a piston driving mechanism for at least pressing the piston member into the held cylinder member, and
a RFID reader for performing wireless communication with the RFID chip on the held cylinder member.

2. The chemical solution injection system according to claim 1,
wherein the distance between centers of the RFID chip and the amplification antenna is generally an integral multiple of the wavelength of the wireless communication.

3. The chemical solution injection system according to claim 2,
wherein the RFID chip and the RFID reader wirelessly communicate at a frequency of 2.45 GHz, and the distance between the centers of the RFID chip and the amplification antenna is approximately 12.5 mm.

4. The chemical solution injection system according to claim 1,
wherein the distance between the centers of the RFID chips and the amplification antenna is 1/2ⁿth (n is a natural number) of a wavelength of the wireless communication.

5. The chemical solution injection system according to any one of claims 1 to 4, wherein, in the chemical solution syringe, a front end portion of the piston member is placed at a rear end portion of the cylinder member, and the RFID chip and the amplification antenna are placed on an outer circumference surface of the rear end portion of the cylinder member, at a position overlapping the front end portion of the piston member.

6. The chemical solution injection system according to any one of claims 1 to 5, wherein the RFID reader includes a communication circuit and a reader antenna connected to the communication circuit, and the reader antenna is placed at the rear of the held cylinder member in the chemical solution injector.

7. The chemical solution injection system according to claim 6,
wherein, in the chemical solution injector, the reader antenna is placed on a plane generally in parallel with a circular plane formed by the amplification antenna of the held cylinder member.

8. The chemical solution injection system according to claim 7,
wherein the reader antenna is formed of a circular polarization antenna.

9. The chemical solution injection system according to claim 7,
wherein the reader antenna is formed of a planar antenna.

10. The chemical solution injection system according to claim 7,
wherein the reader antenna is formed of a linear antenna.

11. The chemical solution injection system according to any one of claims 1 to 10, wherein the chemical solution injector includes display means for outputting with display at least some of various types of data wirelessly received from the RFID chip by the RFID reader.

12. The chemical solution injection system according to any one of claims 1 to 11, wherein the chemical solution injector includes an operation control means for controlling at least the operation of the piston driving mechanism in accordance with various types of data wirelessly received from the RIFD chip by the RFID reader.

13. The chemical solution injection system according to claim 12,
wherein the operation control means allows the operation of the piston driving mechanism, only when the RFID reader detects the RFID chip.

14. The chemical solution injection system according to claim 12 or 13, wherein the operation control means returns the piston driving mechanism to an initial position when completion of the injection operation is detected and then detection of the RFID chip by the RFID reader is ended.

15. The chemical solution injection system according to any one of claims 12 to 14, wherein the operation control means includes a data holding means for holding the various types of data wirelessly received from the RFID chip, and an injection control means for controlling operation of the piston driving mechanism in accordance with at least some of the various types of held data.

16. The chemical solution injection system according to claim 15,
wherein the chemical solution syringe is of a pre-filled type which is shipped with a contrast medium contained therein as the chemical solution to be injected into a patient whose diagnostic image is taken by an imaging diagnostic apparatus, the RFID chip of the chemical solution syringe having data of operation pattern set thereon with which an injection speed of the contrast medium is changed over time, and the operation control means changes an operation speed of the piston driving mechanism in accordance with the operation pattern.

17. The chemical solution injection system according to any one of claims 12 to 16, wherein the operation control means includes a check storing means for storing a predetermined check condition as data, a data comparing means for comparing the check condition stored as data with the various types of data wirelessly received from the RFID chip, and an alarm outputting means for outputting and notifying a check alarm in accordance with the comparison result.

18. The chemical solution injection system according to any one of claims 12 to 17, wherein the RFID chip has at least a production number of the chemical solution syringe set thereon, and the operation control means includes data accumulating means for storing data of the production number of the chemical solution syringe mounted and used to perform injection operation, data comparing means for comparing the stored production number with the new production number, and an alarm outputting means for outputting and notifying a check alarm when the compared production numbers match.

19. The chemical solution injection system according to any one of claims 12 to 18, wherein the RFID chip is put on the chemical solution syringe, to record at least the fact that that chemical solution syringe is once used, and the operation control means includes data recording means for recording, on the RFID chip of the chemical solution syringe, data of the fact that that chemical solution syringe has been mounted and the chemical solution thereof has been injected, and an alarm outputting means for outputting and notifying a check alarm when that data is wirelessly received from the RFID chip of the chemical solution syringe.

20. The chemical solution syringe of the chemical solution injection system according to claim 1, wherein the RFID chip, having a predetermined elongated shape, is wound on the outer circumference surface of the cylinder member, and an amplification antenna having a shape encircling the outer circumference surface of the cylinder member is placed in front of and in parallel with the RFID chip.
